# EUROPEAN PATENT SPECIFICATION

(11) **EP 4 554 493 B1**
(45) Date of publication and mention of the grant of the patent: **03.06.2026**
(21) Application number: 24759223.1
(22) Date of filing: 15.07.2024
(51) Int. Cl.: A61B 17/32, A61B 17/28, A61B 17/00

(54) **SURGICAL INSTRUMENT WITH ELASTOMERIC DISTAL PIPE AND RELATED MANUFACTURING METHODS**
CHIRURGISCHES INSTRUMENT MIT ELASTOMEREM DISTALEM ROHR UND ZUGEHÖRIGEN HERSTELLUNGSVERFAHREN
INSTRUMENT CHIRURGICAL AYANT UN TUYAU DISTAL ÉLASTOMÈRE ET PROCÉDÉS DE FABRICATION ASSOCIÉS

(30) Priority: 17.07.2023 US 202363514011 P; 17.01.2024 US 202418415133
(43) Date of publication of application: 21.05.2025
(73) Proprietor: Cilag GmbH International, 6300 Zug (CH)
(72) Inventor: DANNAHER, William D., Cincinnati, Ohio 45236 (US); STROBL, Geoffrey S., Cincinnati, Ohio 45242 (US); WORRELL, Barry C., Cincinnati, Ohio 45242 (US)
(74) Representative: Carpmaels & Ransford LLP
(86) International application number: PCT/IB2024/056855
(87) International publication number: WO 2025/017460

(56) References cited:
- US-A1- 2009 099 582
- US-A1- 2020 375 619
- US-B2- 10 258 363
- US-B2- 11 324 527
- US-B2- 8 591 536

## Description

### BACKGROUND

A variety of surgical instruments include an end effector having a blade element that vibrates at ultrasonic frequencies to cut and/or seal tissue (e.g., by denaturing proteins in tissue cells). These instruments include piezoelectric elements that convert electrical power into ultrasonic vibrations, which are communicated along an acoustic waveguide to the blade element. The precision of cutting and coagulation may be controlled by the surgeon's technique and adjusting the power level, blade edge, tissue traction and blade pressure.

Examples of ultrasonic surgical instruments include the HARMONIC ACE^{®} Ultrasonic Shears, the HARMONIC WAVE^{®} Ultrasonic Shears, the HARMONIC FOCUS^{®} Ultrasonic Shears, and the HARMONIC SYNERGY^{®} Ultrasonic Blades, all by Ethicon Endo-Surgery, Inc. of Cincinnati, Ohio. Further examples of such devices and related concepts are disclosed in U.S. Pat. No. 5,322,055, entitled "Clamp Coagulator/Cutting System for Ultrasonic Surgical Instruments," issued June 21, 1994; U.S. Pat. No. 5,873,873, entitled "Ultrasonic Clamp Coagulator Apparatus Having Improved Clamp Mechanism," issued February 23, 1999; U.S. Pat. No. 5,980,510, entitled "Ultrasonic Clamp Coagulator Apparatus Having Improved Clamp Arm Pivot Mount," filed October 10, 1997; U.S. Pat. No. 6,325,811, entitled "Blades with Functional Balance Asymmetries for use with Ultrasonic Surgical Instruments," issued December 4, 2001; U.S. Pat. No. 6,773,444, entitled "Blades with Functional Balance Asymmetries for Use with Ultrasonic Surgical Instruments," issued August 10, 2004; and U.S. Pat. No. 6,783,524, entitled "Robotic Surgical Tool with Ultrasound Cauterizing and Cutting Instrument," issued August 31, 2004.

Still further examples of ultrasonic surgical instruments are disclosed in U.S. Pub. No. 2006/0079874, entitled "Tissue Pad for Use with an Ultrasonic Surgical Instrument," published April 13, 2006; U.S. Pub. No. 2007/0191713, entitled "Ultrasonic Device for Cutting and Coagulating," published August 16, 2007; U.S. Pub. No. 2007/0282333, entitled "Ultrasonic Waveguide and Blade," published December 6, 2007; U.S. Pub. No. 2008/0200940, entitled "Ultrasonic Device for Cutting and Coagulating," published August 21, 2008; U.S. Pub. No. 2009/0105750, entitled "Ergonomic Surgical Instruments," published April 23, 2009; U.S. Pub. No. 2010/0069940, entitled "Ultrasonic Device for Fingertip Control," published March 18, 2010; and U.S. Pub. No. 2011/0015660, entitled "Rotating Transducer Mount for Ultrasonic Surgical Instruments," published January 20, 2011; and U.S. Pub. No. 2012/0029546, entitled "Ultrasonic Surgical Instrument Blades," published February 2, 2012.

Some of ultrasonic surgical instruments may include a cordless transducer such as that disclosed in U.S. Pub. No. 2012/0112687, entitled "Recharge System for Medical Devices," published May 10, 2012; U.S. Pub. No. 2012/0116265, entitled "Surgical Instrument with Charging Devices," published May 10, 2012; and/or U.S. Pat. App. No. 61/410,603, filed November 5, 2010, entitled "Energy-Based Surgical Instruments".

Additionally, some ultrasonic surgical instruments may include an articulating shaft section. Examples of such ultrasonic surgical instruments are disclosed in U.S. Pub. No. 2014/0005701, entitled "Surgical Instruments with Articulating Shafts," published January 2, 2014; and U.S. Pub. No. 2014/0114334, entitled "Flexible Harmonic Waveguides/Blades for Surgical Instruments," published April 24, 2014.

US 11 324 527 B2 discloses an end effector assembly comprising a medical forceps having a movable jaw member and an ultrasonic blade whereby a flexible seal is positioned over a proximal portion of the blade and within a distal portion of an inner tube to seal the blade to an inner diameter of the inner tube.

While several surgical instruments and systems have been made and used, it is believed that no one prior to the inventors has made or used the invention described in the appended claims.

### SUMMARY OF THE INVENTION

The present invention provides a surgical instrument as recited in claim 1 and a method of manufacturing a surgical instrument as recited in claim 10. Optional features are recited in the dependent claims.

### BRIEF DESCRIPTION OF THE DRAWINGS

While the specification concludes with claims which particularly point out and distinctly claim this technology, it is believed this technology will be better understood from the following description of certain examples taken in conjunction with the accompanying drawings, in which like reference numerals identify the same elements and in which:
FIG. 1 depicts a perspective view of an exemplary surgical instrument;
FIG. 2 depicts a side elevational view of the end effector of the instrument of FIG. 1, in a closed configuration;
FIG. 3 depicts a perspective view of the end effector of FIG. 2, in an open configuration;
FIG. 4 depicts a perspective view of the end effector of FIG. 2, in a closed configuration;
FIG. 5 depicts an enlarged side elevational view of a shaft assembly configured to be incorporated into the exemplary surgical instrument of FIG. 1;
FIG. 6 depicts an enlarged cross-sectional view of an ultrasonic blade of the shaft assembly taken along line 6-6 of FIG. 5;
FIG. 7 depicts an enlarged cross-sectional view of the shaft assembly of FIG. 5;
FIG. 8 depicts an enlarged side elevational view of a cap affixed to a portion of a sheath configured to be incorporated into the shaft assembly of FIG. 5;
FIG. 9 depicts a sectional view of the cap and the portion of the sheath of FIG. 8;
FIG. 10 depicts an enlarged perspective view of an end effector including an overmolded cap and configured to be incorporated into the surgical instrument of FIG. 1;
FIG. 11 depicts an enlarged perspective view of an ultrasonic blade assembly fitted with a sacrificial seal of the end effector of FIG. 10;
FIG. 12A depicts a sectional view of an ultrasonic blade assembly of FIG. 11; and
FIG. 12B depicts the sectional view of the ultrasonic blade assembly similar to FIG. 12A, but showing the sacrificial seal removed from the end effector.

The drawings are not intended to be limiting in any way, and it is contemplated that various embodiments of the technology may be carried out in a variety of other ways, including those not necessarily depicted in the drawings. The accompanying drawings incorporated in and forming a part of the specification illustrate several aspects of the present technology, and together with the description serve to explain the principles of the technology; it being understood, however, that this technology is not limited to the precise arrangements shown.

### DETAILED DESCRIPTION

The following description of certain examples of the technology should not be used to limit its scope. Other examples, features, aspects, embodiments, and advantages of the technology will become apparent to those skilled in the art from the following description, which is by way of illustration, one of the best modes contemplated for carrying out the technology. As will be realized, the technology described herein is capable of other different and obvious aspects, all without departing from the technology. Accordingly, the drawings and descriptions should be regarded as illustrative in nature and not restrictive.

It is further understood that any one or more of the teachings, expressions, embodiments, examples, etc. described herein may be combined with any one or more of the other teachings, expressions, embodiments, examples, etc. that are described herein. The following-described teachings, expressions, embodiments, examples, etc. should therefore not be viewed in isolation relative to each other. Various suitable ways in which the teachings herein may be combined will be readily apparent to those of ordinary skill in the art in view of the teachings herein.

For clarity of disclosure, the terms "proximal" and "distal" are defined herein relative to a human or robotic operator of the surgical instrument. The term "proximal" refers to the position of an element closer to the human or robotic operator of the surgical instrument and further away from the surgical end effector of the surgical instrument. The term "distal" refers to the position of an element closer to the surgical end effector of the surgical instrument and further away from the human or robotic operator of the surgical instrument.

### I. Exemplary Ultrasonic Surgical Instrument

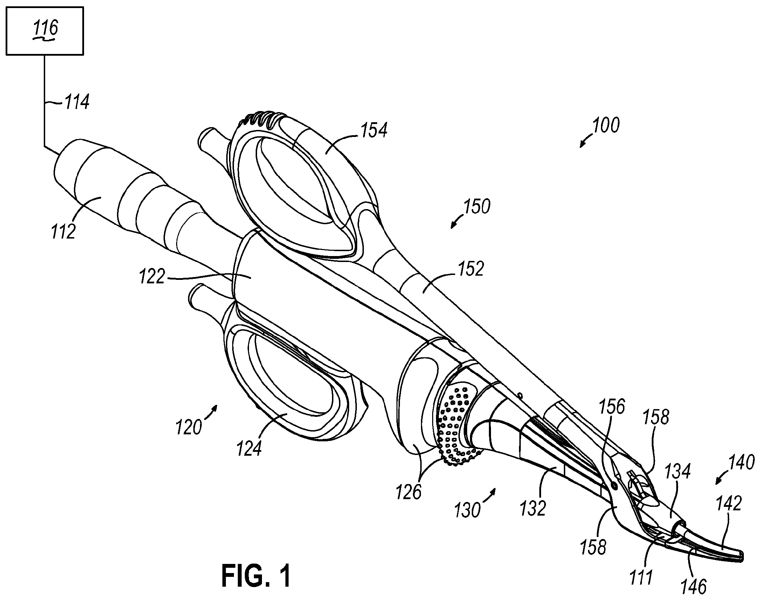

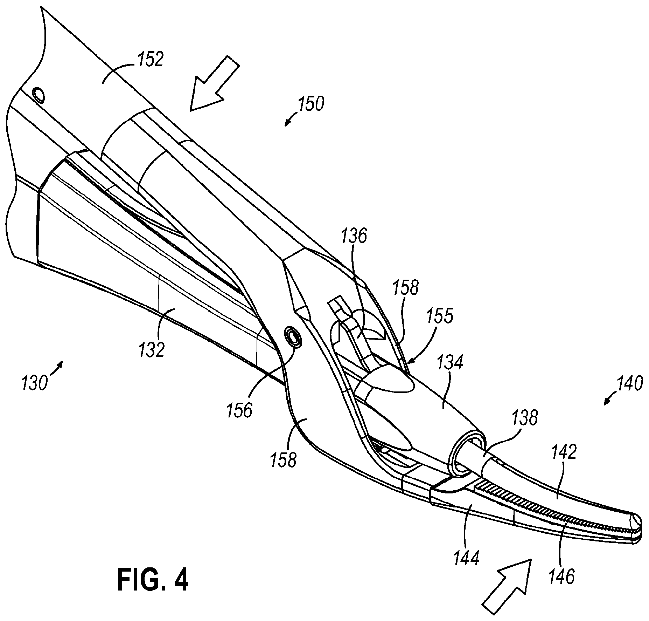

FIGS. 1-4 illustrate an exemplary ultrasonic surgical instrument (100). At least part of each instrument (100) may be constructed and operable in accordance with at least some of the teachings of U.S. Pat. No. 5,322,055; U.S. Pat. No. 5,873,873; U.S. Pat. No. 5,980,510; U.S. Pat. No. 6,325,811; U.S. Pat. No. 6,773,444; U.S. Pat. No. 6,783,524; U.S. Pub. No. 2006/0079874; U.S. Pub. No. 2007/0191713; U.S. Pub. No. 2007/0282333; U.S. Pub. No. 2008/0200940; U.S. Pub. No. 2009/0105750; U.S. Pub. No. 2010/0069940; U.S. Pub. No. 2011/0015660; U.S. Pub. No. 2012/0112687; U.S. Pub. No. 2012/0116265; U.S. Pub. No. 2014/0005701; U.S. Pub. No. 2014/0114334; U.S. Pat. App. No. 61/410,603; and/or U.S. Pat. App. No. 14/028,717. As described therein and as will be described in greater detail below, instrument (100) is operable to cut tissue and seal or weld tissue (e.g., a blood vessel, etc.) substantially simultaneously. It should also be understood that instrument (100) may have various structural and functional similarities with the HARMONIC ACE^{®} Ultrasonic Shears, the HARMONIC WAVE^{®} Ultrasonic Shears, the HARMONIC FOCUS^{®} Ultrasonic Shears, and/or the HARMONIC SYNERGY^{®} Ultrasonic Blades. Furthermore, instrument (100) may have various structural and functional similarities with the devices taught in any of the other references that are cited herein.

To the extent that there is some degree of overlap between the teachings of the references cited herein, the HARMONIC ACE^{®} Ultrasonic Shears, the HARMONIC WAVE^{®} Ultrasonic Shears, the HARMONIC FOCUS^{®} Ultrasonic Shears, and/or the HARMONIC SYNERGY^{®} Ultrasonic Blades, and the following teachings relating to instruments (100), there is no intent for any of the description herein to be presumed as admitted prior art. Several teachings herein will in fact go beyond the scope of the teachings of the references cited herein and the HARMONIC ACE^{®} Ultrasonic Shears, the HARMONIC WAVE^{®} Ultrasonic Shears, the HARMONIC FOCUS^{®} Ultrasonic Shears, and the HARMONIC SYNERGY^{®} Ultrasonic Blades.

FIG. 1 illustrates an exemplary ultrasonic surgical instrument (100) that is configured to be used in open surgical procedures. Instrument (100) of this example comprises a handle assembly (120), a shaft assembly (130), and an end effector (140). Handle assembly (120) comprises a body (122) including a finger grip ring (124) and a pair of buttons (126). Instrument (100) also includes a clamp arm assembly (150) that is pivotable toward and away from body (122). Clamp arm assembly (150) includes a shank (152) with a thumb grip ring (154). Thumb grip ring (154) and finger grip ring (124) together provide a scissor grip type of configuration. It should be understood, however, that various other suitable configurations may be used, including but not limited to a pistol grip configuration.

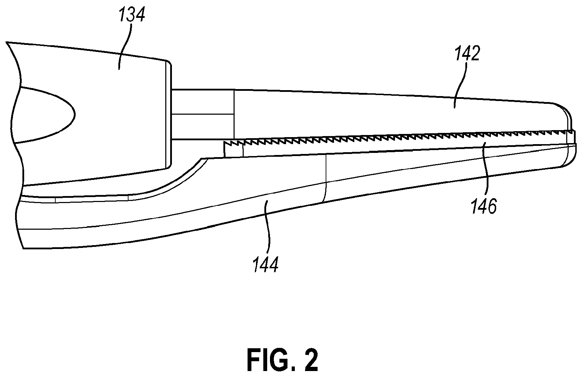

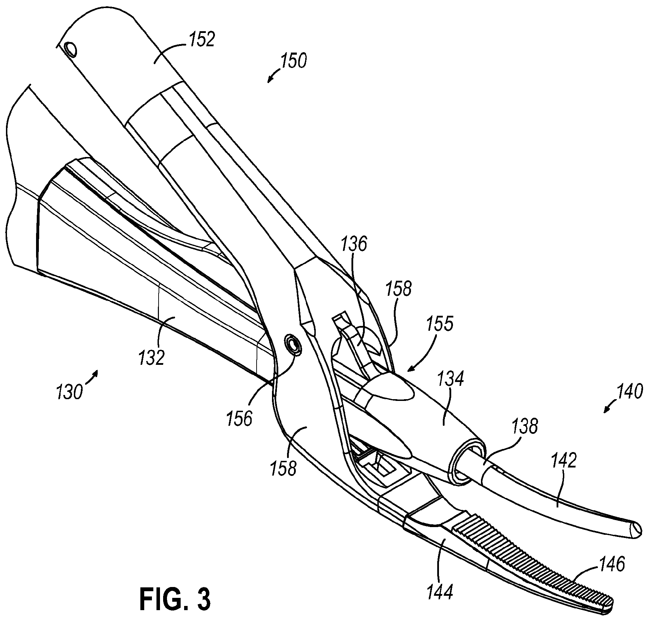

Shaft assembly (130) comprises an outer sheath (132) extending distally from body (122). A cap (134) is secured to the distal end of sheath (132). As best seen in FIGS. 2-4, end effector (140) comprises an ultrasonic blade (142) and a clamp arm (144). Ultrasonic blade (142) extends distally from cap (134). Clamp arm (144) is an integral feature of clamp arm assembly (150). Clamp arm (144) includes a clamp pad (146) facing ultrasonic blade (142). Clamp arm assembly (150) is pivotally coupled with outer sheath (132) via a pin (156). Clamp arm (144) is positioned distal to pin (156); while shank (152) and thumb grip ring (154) are positioned proximal to pin (156). Thus, as shown in FIGS. 3-4, clamp arm (144) is pivotable toward and away from ultrasonic blade (142) based on pivoting of thumb grip ring (154) toward and away from body (122) of handle assembly (120). It should therefore be understood that an operator may squeeze thumb grip ring (154) toward body (122) to thereby clamp tissue between clamp pad (146) and ultrasonic blade (142) to transect and/or seal the tissue. In some versions, one or more resilient members are used to bias clamp arm (144) to the open position shown in FIG. 3. By way of example only, such a resilient member may comprise a leaf spring, a torsion spring, and/or any other suitable kind of resilient member.

Referring back to FIG. 1, an ultrasonic transducer assembly (112) extends proximally from body (122) of handle assembly (120). Transducer assembly (112) is coupled with a generator (116) via a cable (114). Transducer assembly (112) receives electrical power from generator (116) and converts that power into ultrasonic vibrations through piezoelectric principles. Generator (116) may include a power source and control module that is configured to provide a power profile to transducer assembly (112) that is particularly suited for the generation of ultrasonic vibrations through transducer assembly (112). By way of example only, generator (116) may comprise a GEN 300 sold by Ethicon Endo-Surgery, Inc. of Cincinnati, Ohio. In addition or in the alternative, generator (116) may be constructed in accordance with at least some of the teachings of U.S. Pub. No. 2011/0087212, entitled "Surgical Generator for Ultrasonic and Electrosurgical Devices," published April 14, 2011. It should also be understood that at least some of the functionality of generator (116) may be integrated into handle assembly (120), and that handle assembly (120) may even include a battery or other on-board power source such that cable (114) is omitted. Still other suitable forms that generator (116) may take, as well as various features and operabilities that generator (116) may provide, will be apparent to those of ordinary skill in the art in view of the teachings herein.

Ultrasonic vibrations that are generated by transducer assembly (112) are communicated along an acoustic waveguide (138), which extends through shaft assembly (130) to reach ultrasonic blade (142). Waveguide (138) is secured within shaft assembly (130) via a pin (not shown), which passes through waveguide (138) and shaft assembly (130). This pin is located at a position along the length of waveguide (138) corresponding to a node associated with resonant ultrasonic vibrations communicated through waveguide (138). As noted above, when ultrasonic blade (142) is in an activated state (i.e., vibrating ultrasonically), ultrasonic blade (142) is operable to effectively cut through and seal tissue, particularly when the tissue is being clamped between clamp pad (146) and ultrasonic blade (142). It should be understood that waveguide (138) may be configured to amplify mechanical vibrations transmitted through waveguide (138). Furthermore, waveguide (138) may include features operable to control the gain of the longitudinal vibrations along waveguide (138) and/or features to tune waveguide (138) to the resonant frequency of the system.

In the present example, the distal end of ultrasonic blade (142) is located at a position corresponding to an anti-node associated with resonant ultrasonic vibrations communicated through waveguide (138), in order to tune the acoustic assembly to a preferred resonant frequency fₒ when the acoustic assembly is not loaded by tissue. When transducer assembly (112) is energized, the distal end of ultrasonic blade (142) is configured to move longitudinally in the range of, for example, approximately 10 to 500 microns peak-to-peak, and in some instances in the range of about 20 to about 200 microns at a predetermined vibratory frequency fₒ of, for example, 55.5 kHz. When transducer assembly (112) of the present example is activated, these mechanical oscillations are transmitted through the waveguide to reach ultrasonic blade (102), thereby providing oscillation of ultrasonic blade (102) at the resonant ultrasonic frequency. Thus, when tissue is secured between ultrasonic blade (142) and clamp pad (46), the ultrasonic oscillation of ultrasonic blade (142) may simultaneously sever the tissue and denature the proteins in adjacent tissue cells, thereby providing a coagulative effect with relatively little thermal spread. In some versions, an electrical current may also be provided through ultrasonic blade (142) and/or clamp pad (146) to also seal the tissue.

An operator may activate buttons (126) to selectively activate transducer assembly (112) to thereby activate ultrasonic blade (142). In the present example, two buttons (126) are provided - one for activating ultrasonic blade (142) at a low power and another for activating ultrasonic blade (142) at a high power. However, it should be understood that any other suitable number of buttons and/or otherwise selectable power levels may be provided. For instance, a foot pedal may be provided to selectively activate transducer assembly (112). Buttons (126) of the present example are positioned such that an operator may readily fully operate instrument (100) with a single hand. For instance, the operator may position their thumb in thumb grip ring (154), position their ring finger in finger grip ring (124), position their middle finger about body (122), and manipulate buttons (126) using their index finger. Of course, any other suitable techniques may be used to grip and operate instrument (100); and buttons (126) may be located at any other suitable positions.

The foregoing components and operabilities of instrument (100) are merely illustrative. Instrument (100) may be configured in numerous other ways as will be apparent to those of ordinary skill in the art in view of the teachings herein. By way of example only, at least part of instrument (100) may be constructed and/or operable in accordance with at least some of the teachings of any of the following: U.S. Pat. No. 5,322,055; U.S. Pat. No. 5,873,873; U.S. Pat. No. 5,980,510; U.S. Pat. No. 6,325,811; U.S. Pat. No. 6,783,524; U.S. Pub. No. 2006/0079874; U.S. Pub. No. 2007/0191713; U.S. Pub. No. 2007/0282333; U.S. Pub. No. 2008/0200940; U.S. Pub. No. 2010/0069940; U.S. Pub. No. 2011/0015660; U.S. Pub. No. 2012/0112687; U.S. Pub. No. 2012/0116265; U.S. Pub. No. 2014/0005701; U.S. Pub. No. 2014/0114334; and/or U.S. Pat. App. No. 14/031,665. Additional merely illustrative variations for instrument (100) will be described in greater detail below. It should be understood that the below described variations may be readily applied to instrument (100) described above and any of the instruments referred to in any of the references that are cited herein, among others.

### II. Ultrasonic Surgical Instrument with Distal Overmolded Features

In some instances, it may be desirable to provide a version of ultrasonic surgical instrument (100) with a cap (2034, 2134) formed by overmolding. Overmolding is an injection molding technique where an elastomer, such as silicone, is injected over a substrate layer of material. Overmolding generally reduces costly assembly and provides flexible features to otherwise rigid components. Cap (2034, 2134) prevents proximal section of an ultrasonic blade (2042) from interfering with clamp arm assembly (150) during closure. Cap (2034, 2134) also acts as a tissue stop and is spaced apart from the ultrasonic blade assembly (2042) to prevent inadvertent contact between the ultrasonic blade assembly (2042) and the cap (2034, 2134). Utilizing overmolding to form cap (2034, 2134) provides an advantage of cost savings and eases assembly while providing the functions of cap (2034, 2134).

### A. Cap Overmolded upon an Ultrasonic Blade

In some instances, and in accordance with the present claimed invention, it may be desirable to provide a version of a cap (2034) in the form of a distal pipe, that is overmolded directly upon an ultrasonic blade assembly (2041). In this respect, any cap discussed herein may also be referred to as a "distal pipe." As explained above, the substrate material in this version is ultrasonic blade assembly (2041) and overmolded portion is cap (2034). Having cap (2034) directly overmolded upon ultrasonic blade assembly (2041) provides the advantage of cost savings while producing a clinically equivalent or better performing elastomeric distal portion of sheath (2032).

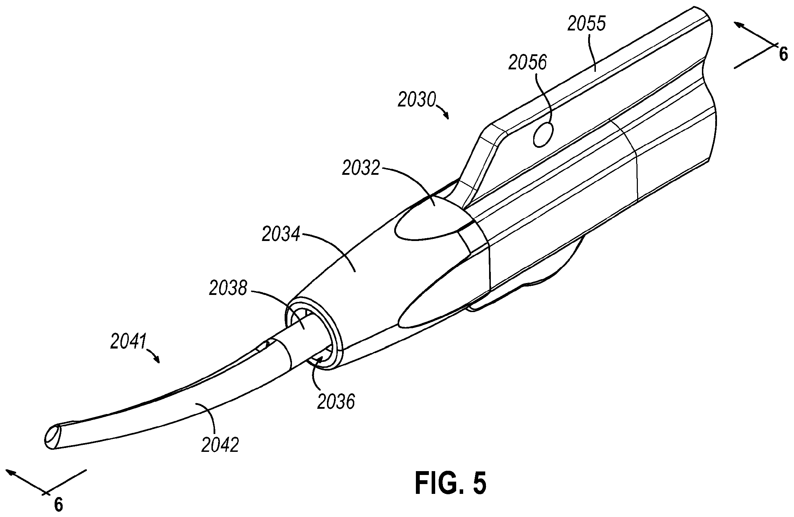

FIG. 5 depicts a shaft assembly (2030) similar to shaft assembly (130) that is configured to be integrated into ultrasonic surgical instrument (100). Shaft assembly (2030) includes cap (2034), sheath (2032), and ultrasonic blade assembly (2041), similar to that of shaft assembly (130) described above. Shaft assembly (2030) differs from shaft assembly (130), because cap (2034) is directly overmolded upon ultrasonic blade assembly (2041). Cap (2034) includes an overmolding material having properties such as CC4 rating, temperature resistance, and regulatorily compliant material. Cap (2034) more specifically may be constructed of a silicone or other synthetic elastomer that displays the properties both of viscosity in a flowable form before hardening and elasticity once cured. Cap (2034) includes a hollow interior (2036) and a tapered exterior region (2039). Hollow interior (2036) is configured to allow ultrasonic blade (2042) to pass through cap (2034). Tapered exterior region (2039) is configured to allow freedom of movement during use regarding the distal portions of clamp arm assembly (150) so that neither ultrasonic blade assembly nor clamp arm (144) engage cap (2034) during use. As shown in the assembled state, cap (2034) extends distally from sheath (2032) and ultrasonic blade (2042) extends distally from within hollow interior (2036). In the present version, sheath (2032) is constructed of thermoplastic vulcanizates (TPV) including, but not limited to, polypropylene and EPDM. In some versions, sheath (2032) may be formed of a surgically safe metal. Sheath (2032) includes a tubular shape and a raised rib (2055), which may also be referred to as a "fin." Raised rib (2055) defines a pin bore (2056) configured to accept a pin (56) to attach clamp arm assembly (150).

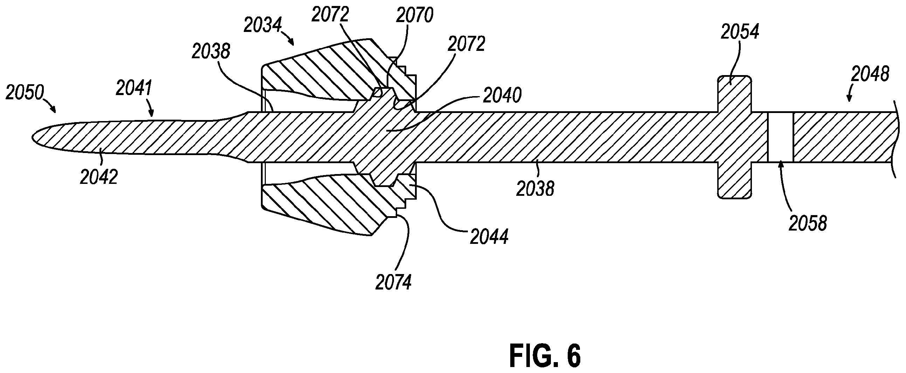

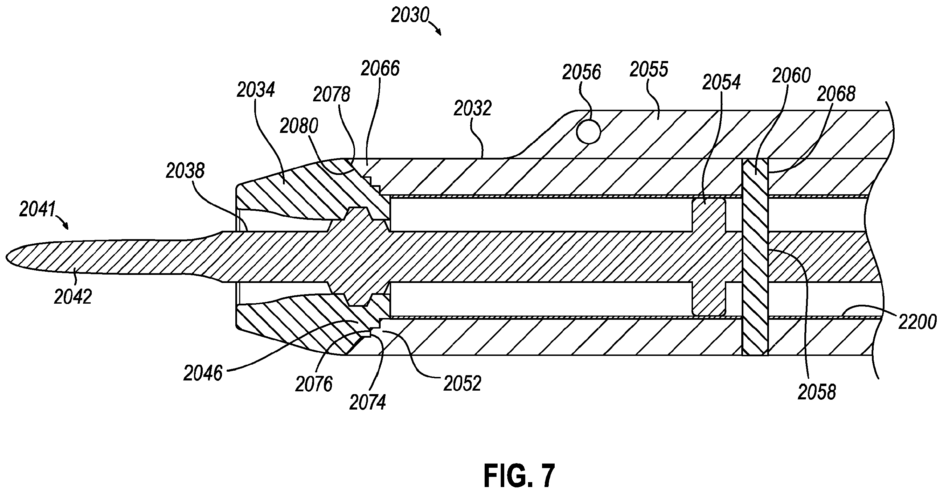

FIG. 6 depicts ultrasonic blade assembly (2041) overmolded with cap (2034). Ultrasonic blade assembly (2041) includes ultrasonic blade (2042) and waveguide (2038) similar in form and construction to ultrasonic blade (142) and waveguide (138) unless otherwise discuss below. Cap (2034) is formed by injecting a liquid or aqueous resin such as a silicone, plastic, or rubber within a mold. The mold retains a portion or all of ultrasonic blade assembly (2041) and defines the shape of cap (2034) until the liquid or aqueous resin solidifies. Once the resin solidifies, ultrasonic blade assembly (2041) is removed from the mold. Cap (2034) may be positioned upon ultrasonic blade (2042) or waveguide (2038). As shown, cap (2034) is overmolded upon waveguide (2038). Ultrasonic blade assembly (2041) extends distally from a proximal portion (2048) to a distal portion (2050). While ultrasonic blade assembly (2041) may be formed of multiple components, ultrasonic blade assembly (2041) may alternatively be singularly and unitarily formed. Proximal portion (2048) of ultrasonic blade assembly (2041) is operably secured to an ultrasonic transducer (112) as discussed above. Proximal portion (2048) defines an insulated pin bore (2058) configured to secure ultrasonic blade assembly (2041) to sheath (2032) *(see* FIG. 7) by an insulated pin (2060) *(see* FIG. 7).

Ultrasonic blade assembly (2041) further includes one or more first mating features (2040) positioned between proximal and distal portions (2048, 2050) having one or more raised, recessed, or stepped portions. First mating feature (2040) is positioned upon waveguide (2038). First mating feature (2040) is configured to provide a stronger bond with cap (2034), such as by additional surface area contact and mechanical overlap in the longitudinal direction. As shown, first mating feature (2040) extends around the circumference of ultrasonic blade assembly (2041). A second mating feature (2044) of cap (2034) is formed where cap (2034) engages first mating feature (2040) during the overmolding process that complements first mating feature (2040).

More particularly, first mating feature (2040) is in the form of an annular, central rib (2070), and two additional annular, side ribs (2072) that are all raised above a nominal outside diameter of waveguide (2038). Central rib (2070) includes a central outside diameter that is greater than a side outside diameter of side ribs (2072). Second mating feature (2044) includes an annular channel that corresponds with central rib (2070) and is also bonded to side ribs (2072). Side ribs (2072) space apart cap (2034) from waveguide (2038).

In other versions, first mating feature (2040) may include one or more of the following features: a key, a keyway, a post, a recess, a channel or any mating feature apparent to a person skilled in the art known to increase a bond between a rigid component and a molded component. In yet other versions, a plurality of discrete first mating features (2040) may be aligned or staggered around a circumference of waveguide (2038). In still yet other versions, cap (2034) may be directly overmolded to ultrasonic blade assembly (2041) without any mating features.

As shown in FIGS. 6 and 7, cap (2034) further includes a third mating feature (2074) positioned upon proximal exterior of cap (2034). Third mating feature (2074) is configured to be mated with a distal portion of sheath (2032). Third mating feature (2074) includes one or more raised, recessed, tapered, or stepped portions. As shown, third mating feature (2074) includes annular raised rings that extends around a circumference of cap (2034). These rings are arranged in multiple steps that decrease in diameter in a proximal direction. Distal portion of sheath (2032) includes a fourth mating feature (2076) that complements third mating feature (2074) to produce a liquid tight seal between cap (2034) and sheath (2032). Third mating feature (2074) may be constructed with the aid of a mold that corresponds with the shape of fourth mating feature (2076) before inserting ultrasonic blade assembly (2041) within sheath (2032). In other versions, cap (2034) may be simultaneously overmolded over ultrasonic blade assembly (2041) as well as directly upon fourth mating feature (2076) after sheath (2032) has been formed within a mold.

It should be noted, raised, recessed, tapered or stepped portions may be reversed on first and third mating features (2040, 2074) relative to second and fourth mating features (2044, 2076) respectively. For example rather than raised portions (ribs) being positioned on first mating feature (2040), first mating feature (2040) would include recessed portions and second mating feature (2044) would include raised portions. As shown, first and second mating features (2040, 2044) are annular with a rectangular profile but first and second mating features (2040, 2044) take any other shape known in the art to facilitate a strong bond between two mating portions, such as a triangular, a dovetail, or a round shape.

An additional support (2054) is formed by overmolding on an exterior surface of waveguide (2038) between second mating feature (2044) and proximal portion (2048) of ultrasonic blade assembly (2041). Support (2054) includes an annular shape and is configured to extend radially from waveguide (2038) and engage an inner surface of sheath (2032) (*see* FIG. 7). Support (2054) is positioned to inhibit energy from being transferred from ultrasonic blade assembly (2041) to sheath (2032) and further via sheath (2032) to the user. Support (2054) in one version is molded at the same time as cap (2034) and before installing ultrasonic blade assembly (2041) within sheath (2032). In one version, support (2054) and cap (2034) are molded separately. In one version, support (2054) may be omitted entirely and/or sheath (2032) may be configured to provide a mechanical ground for ultrasonic blade assembly (2041).

FIG. 7 depicts ultrasonic blade assembly (2041) positioned within sheath (2032). Sheath (2032) includes a sheath bore (2068) configured to receive insulated pin (2060). Sheath (2032) further includes a distally positioned fourth mating feature (2076). Third and fourth mating features (2074, 2076) are constructed similarly to first and second mating features (2040, 2044) and include raised and recessed portions (2046, 2052) that engage one another. Third and fourth mating features (2074, 2076) further include complementary tapered sections (2078, 2080) configured to complement one another. In some versions, sheath (2032) may be shorter than sheath (132) to compensate for third and fourth mating features (2074, 2076). All mating features (2040, 2044, 2074, 2076) of the present version are configured to inhibit debris, such as liquid or solids, from entering an interior of surgical instrument (100) from an external environment so that surgical instrument (100) may be easily cleaned and/or sterilized.

During assembly, sheath (2032) is translated distally over support (2054). Support (2054) frictionally engages an inner surface of sheath (2043) until fourth mating feature (2076) meshes or engages with third mating feature (2074) aligning sheath bore (2068) with insulated pin bore (2058). Once third and fourth mating features (2074, 2076) engage one another, insulated pin (2060) is inserted within sheath bore (2068) and further through insulated pin bore (2058) to locate and secure proximal end of sheath (2032) relative to waveguide (2038). Insulated pin (2060) inhibits transmitting ultrasonic energy to sheath (2032). Support (2054) also is configured to provide support without transmitting ultrasonic energy to sheath (2032). In some versions, more than one support (2054) and more than one insulated pin (2060) may be used to stabilize surgical instrument. Support (2054) and/or insulated pin (2060) may be placed at a node associated with resonant ultrasonic vibrations communicated through waveguide (2038). Alternatively, sheath (2032) may be overmolded over cap (2034) and support (2054) or vice versa.

### B. Overmolded Cap with Locking Feature

In some instances, it may be desirable to provide a version of a cap (2134) that is overmolded directly upon a sheath (2132). As explained above regarding the overmolding process, the substrate in this version is sheath (2132) and overmolded portion is cap (2034). Cap (2034) is directly overmolded upon sheath (2132) and provides the advantage of cost savings while producing a clinically equivalent or better performing elastomeric distal portion of sheath (2132).

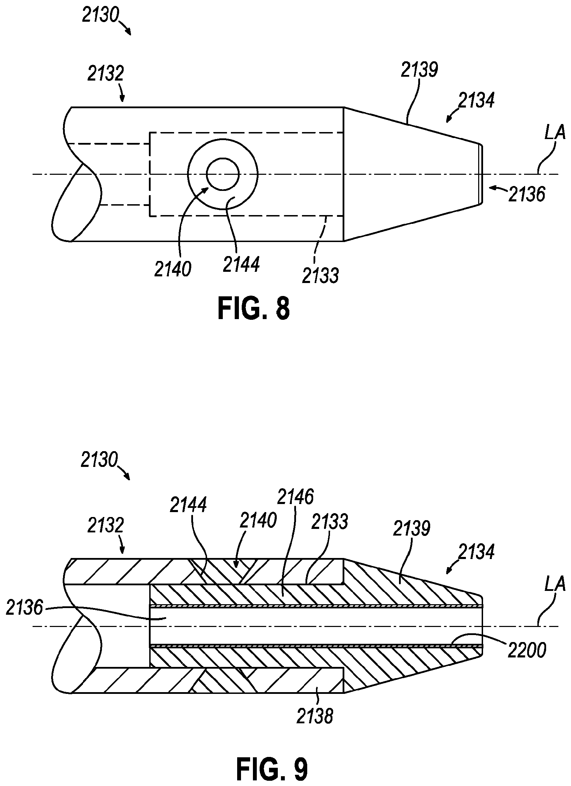

FIGS. 8 and 9 depict a portion of shaft assembly (2130) similar in form and construction to shaft assembly (2030) unless otherwise discussed below. Shaft assembly (2130) differs from shaft assembly (2030) in that shaft assembly (2130) includes a cap (2134) that is overmolded upon a sheath (2132) rather than cap (2134) being overmolded upon ultrasonic blade assembly (2041). Sheath (2132) is similar in form and construction to sheath (2032) unless otherwise discussed below. Sheath (2132) extends distally along a longitudinal axis (LA) to cap (2134). Sheath (2132) includes a hollow tube (2138) that defines a pair of sheath mating features (2144) that extend transversely relative to longitudinal axis (LA). Each sheath mating feature (2144) of the present version is in the form of a countersunk bore.

Cap (2134) includes a distally positioned tapered exterior (2139), a hollow interior (2136), a proximal tube (2146), and a pair of cap mating features (2140). Cap (2134), unlike cap (2034), includes a proximal tube (2146) that extends within an inner surface (2133) of sheath (2132). Proximal tube (2146) is sized to fit within sheath (2132). Proximal tube (2146) provides additional protection from a deflected ultrasonic blade assembly upon inadvertently engaging with sheath (2132). Pair of cap mating features (2140) extend transversely within pair of sheath mating features (2144) from proximal tube (2146). Hollow tube (2138) encircles proximal tube (2146). Each sheath mating feature (2144) extends transversely away from longitudinal axis (LA) through opposing sides of hollow tube (2138). Mating features (2140, 2144) are configured to prevent debris, such as liquid or solids, from entering hollow tube (2138) and retain cap (2034) to a distal portion of sheath (2132). In the present version, sheath mating features (2144) include a dovetail shape that defines cap mating feature (2140), which affixes sheath mating feature (2144) to cap mating feature (2140). In other words, cap mating feature (2140) includes a countersunk bore with progressively decreasing diameter as it approaches longitudinal axis (LA). These counter sunk bores are created by molding or machining, but it will be appreciated that other methods of manufacture known in the art may be similarly used to form countersunk bores. Sheath mating features (2144) and additional molds or retainers are used to direct a liquid or aqueous resin into sheath mating features (2144) to form cap mating features (2140) as well as remaining portion of cap (2134). After cap mating features (2140) has cured, ultrasonic blade assembly (2041) *(see* FIG. 5) is inserted within shaft assembly (2030). In other versions, ultrasonic blade assembly (2041) *(see* FIG. 5) is inserted within sheath (2132) before cap (2134) is overmolded.

### C. Method of Lubricating Cap of Ultrasonic Surgical Instrument

In some instances, reducing assembly forces due to friction of an ultrasonic blade passing through an elastomer cap may be advantageous. This may be completed before assembling the components, or by assembling with a cap formed from internally lubricated materials. In this respect, components may be lubricated before assembly with one or more medical grade lubricants, such as sodium stearate, silicone fluid, or any medically safe lubricant known in the art. Alternatively, the materials for cap could be one that is self-lubricating, such as NUSIL^{®} MED 14855 Liquid Silicone Rubber, a self-lubricating injection molding elastomer with high lubrication properties. Overmolding with a self-lubricating material provides the advantage of reducing steps of lubricating components before assembly and a lubricant (2200) provided from the self-lubricating material acts as a barrier between cap and ultrasonic blade when ultrasonic blade (2042) is deflected causing inadvertent contact.

Referring back, FIG. 6 shows the ultrasonic blade assembly (2041) before installation within sheath (2032), whereas FIG. 7 shows ultrasonic blade assembly (2041) after installation within sheath (2032). Support (2054), cap (2034), and/or corresponding area of sheath (2032) is lubricated with a lubricant (2200) before ultrasonic blade assembly (2041) is installed within sheath (2032). In other versions, cap (2034) and support (2054) *(see* FIG. 6) are constructed with a self-lubricating elastomer reducing the assembly steps by the pre-assembly lubrication step.

FIGS. 8 and 9 depict a portion of shaft assembly (2130) similar to shaft assembly (2030) extending distally along a longitudinal axis (LA) before installing ultrasonic blade assembly (2041). Cap (2034) and/or corresponding area of sheath (2032) is lubricated with a lubricant (2200) before ultrasonic blade assembly (2041) is installed within sheath (2132). In other versions, cap (2134) is constructed of a self-lubricating elastomer reducing the assembly steps by the pre-assembly lubrication step.

### D. Method of Forming a Cap with a Sacrificial Seal

In some instances, it may be advantageous to place an elastomeric sacrificial seal (2370) that is coated with a surfactant (2372) to prevent overmolding material from adhering to an undesirable surface, such as an ultrasonic node.

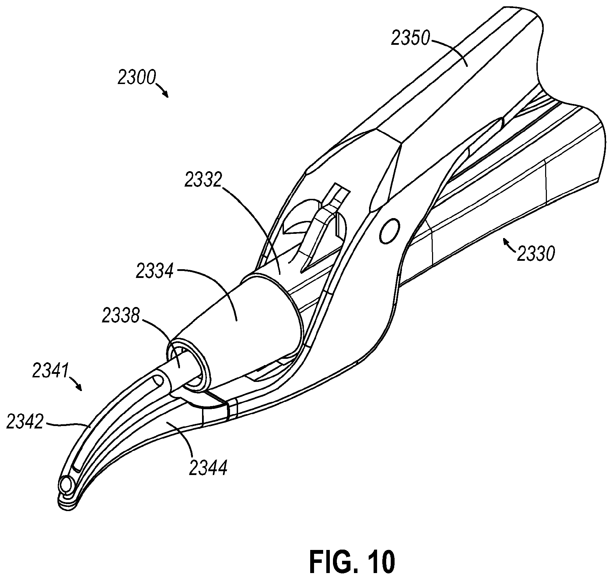

To this end, FIG. 10 depicts an end effector (2300) configured to be incorporated into surgical instrument (100). End effector (2300) is similar in form and construction to end effector (140) and includes a shaft assembly (2330) and a clamp arm assembly (2350) unless otherwise discussed below. Shaft assembly (2330) includes a sheath (2332) and an ultrasonic blade assembly (2341). Clamp arm assembly (2350) has a clamp arm (2344) and is pivotally coupled to sheath (2332) to facilitate the movement of clamp arm (2344) toward and away from ultrasonic blade (2314).

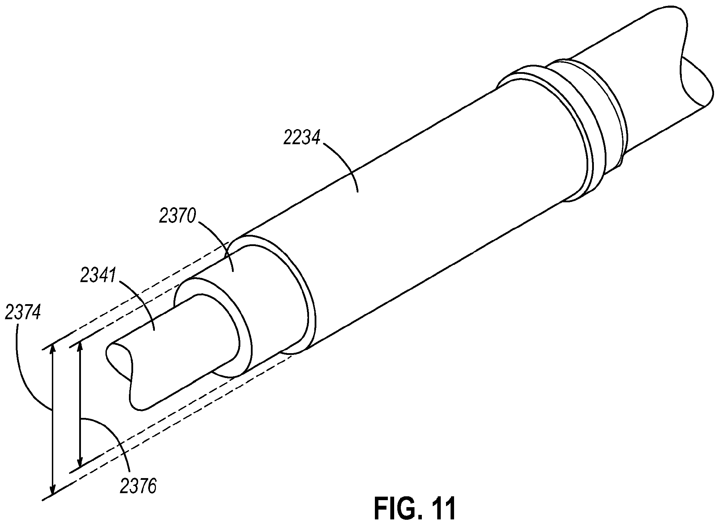

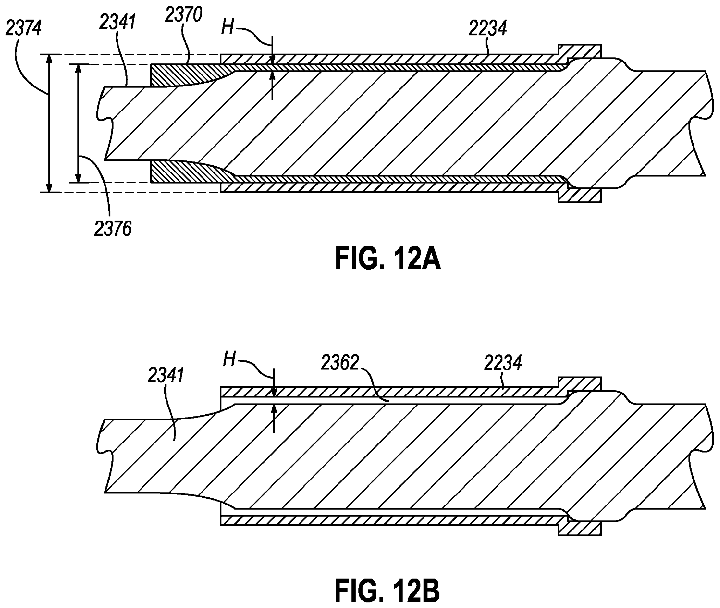

FIGS. 11-12B depict ultrasonic blade assembly (2341) with sacrificial seal (2370) installed between cap (2234) and ultrasonic blade assembly (2341). Sacrificial seal (2370) is constructed of materials having elastomeric properties including, but not limited to, silicone rubber, foam, latex, nylon, and any other material known in the art to provide a spacing between an overmolded material and substrate material. Sacrificial seal (2370) includes a tubular shape having an outside diameter (2374) and an inside diameter (2376). The distance between outside diameter (2374) and inside diameter (2376) includes a predetermined height (H) that is maintained by the presence of sacrificial seal (2370) during the overmolding process. Before installing sacrificial seal (2370) over ultrasonic blade assembly (2341), ultrasonic blade assembly (2341) may be coated with a surfactant (2372) to inhibit binding of sacrificial seal (2370) upon ultrasonic blade assembly (2341) or cap (2234) that is formed by overmolding. Sacrificial seal (2370) is then installed by passing ultrasonic blade assembly (2341) through a bore of sacrificial seal (2370). Next, ultrasonic blade assembly (2341) and sacrificial seal (2370) are placed within a mold to further define exterior profile of cap (2234). In one version, cap (2234) is overmolded upon sheath (2032) *(see* FIG. 6-7). In one version, cap (2234) is overmolded within sheath (2132) *(see* FIG. 8-9). Sacrificial seal (2370) may be placed a set distance within sheath (2032, 2132) to produce third mating surface (2074) *(see* FIG. 7) or proximal tube (2146) *(see* FIG. 8). Sacrificial seal (2370) is also positioned about ultrasonic blade assembly (2341). A mold is placed around sacrificial seal (2370) with a gap (2362) configured to form cap (2234) by overmolding. It should be noted that additional molds, constraints, or sacrificial seals are positioned around cap (2234) to further define, shape, or contour cap (2234). Once overmolding material of cap (2234) has cured, sacrificial seal (2370) is removed and discarded. Thus, sacrificial seal (2370) defines a gap (2362) between ultrasonic blade assembly (2341) and cap (2234) to inhibit contact of ultrasonic blade assembly (2341) with cap (2234) when ultrasonic blade assembly (2341) is inadvertently deflected.

### IV. Miscellaneous

It should be understood that any one or more of the teachings, expressions, embodiments, examples, etc. described herein may be combined with any one or more of the other teachings, expressions, embodiments, examples, etc. that are described herein. The above-described teachings, expressions, embodiments, examples, etc. should therefore not be viewed in isolation relative to each other. Various suitable ways in which the teachings herein may be combined will be readily apparent to those of ordinary skill in the art in view of the teachings herein.

Versions of the devices described above may have application in conventional medical treatments and procedures conducted by a medical professional, as well as application in robotic-assisted medical treatments and procedures. By way of example only, various teachings herein may be readily incorporated into a robotic surgical system such as the DAVINCI^{™} system by Intuitive Surgical, Inc., of Sunnyvale, California. Similarly, those of ordinary skill in the art will recognize that various teachings herein may be readily combined with various teachings of any of the following: U.S. Pat. No. 5,792,135, entitled "Articulated Surgical Instrument For Performing Minimally Invasive Surgery With Enhanced Dexterity and Sensitivity," issued Aug. 11, 1998; U.S. Pat. No. 5,817,084, entitled "Remote Center Positioning Device with Flexible Drive," issued Oct. 6, 1998; U.S. Pat. No. 5,878,193, entitled "Automated Endoscope System for Optimal Positioning," issued March 2, 1999; U.S. Pat. No. 6,231,565, entitled "Robotic Arm DLUS for Performing Surgical Tasks," issued May 15, 2001; U.S. Pat. No. 6,783,524, entitled "Robotic Surgical Tool with Ultrasound Cauterizing and Cutting Instrument," issued Aug. 31, 2004; U.S. Pat. No. 6,364,888, entitled "Alignment of Master and Slave in a Minimally Invasive Surgical Apparatus," issued April 2, 2002; U.S. Pat. No. 7,524,320, entitled "Mechanical Actuator Interface System for Robotic Surgical Tools," issued April 28, 2009; U.S. Pat. No. 7,691,098, entitled "Platform Link Wrist Mechanism," issued April 6, 2010; U.S. Pat. No. 7,806,891, entitled "Repositioning and Reorientation of Master/Slave Relationship in Minimally Invasive Telesurgery," issued Oct. 5, 2010; U.S. Pat. No. 8,844,789, entitled "Automated End Effector Component Reloading System for Use with a Robotic System," issued Sept. 30, 2014; U.S. Pat. No. 8,820,605, entitled "Robotically-Controlled Surgical Instruments," issued Sept. 2, 2014; U.S. Pat. No. 8,616,431, entitled "Shiftable Drive Interface for Robotically-Controlled Surgical Tool," issued Dec. 31, 2013; U.S. Pat. No. 8,573,461, entitled "Surgical Stapling Instruments with Cam-Driven Staple Deployment Arrangements," issued Nov. 5, 2013; U.S. Pat. No. 8,602,288, entitled "Robotically-Controlled Motorized Surgical End Effector System with Rotary Actuated Closure Systems Having Variable Actuation Speeds," issued Dec. 10, 2013; U.S. Pat. No. 9,301,759, entitled "Robotically-Controlled Surgical Instrument with Selectively Articulatable End Effector," issued April 5, 2016; U.S. Pat. No. 8,783,541, entitled "Robotically-Controlled Surgical End Effector System," issued July 22, 2014; U.S. Pat. No. 8,479,969, entitled "Drive Interface for Operably Coupling a Manipulatable Surgical Tool to a Robot," issued July 9, 2013; U.S. Pat. Pub. No. 8,800,838, entitled "Robotically-Controlled Cable-Based Surgical End Effectors," issued Aug. 12, 2014; and/or U.S. Pat. No. 8,573,465, entitled "Robotically-Controlled Surgical End Effector System with Rotary Actuated Closure Systems," issued Nov. 5, 2013.

Versions of the devices described above may be designed to be disposed of after a single use, or they can be designed to be used multiple times. Versions may, in either or both cases, be reconditioned for reuse after at least one use. Reconditioning may include any combination of the steps of disassembly of the device, followed by cleaning or replacement of particular pieces, and subsequent reassembly. In particular, some versions of the device may be disassembled, and any number of the particular pieces or parts of the device may be selectively replaced or removed in any combination. Upon cleaning and/or replacement of particular parts, some versions of the device may be reassembled for subsequent use either at a reconditioning facility, or by a clinician immediately prior to a procedure. Those skilled in the art will appreciate that reconditioning of a device may utilize a variety of techniques for disassembly, cleaning/replacement, and reassembly. Use of such techniques, and the resulting reconditioned device, are all within the scope of the present application.

By way of example only, versions described herein may be sterilized before and/or after a procedure. In one sterilization technique, the device is placed in a closed and sealed container, such as a plastic or TYVEK bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, or steam.

Having shown and described various embodiments of the present invention, further adaptations of the methods and systems described herein may be accomplished by appropriate modifications by one of ordinary skill in the art without departing from the scope of the present invention. Several of such potential modifications have been mentioned, and others will be apparent to those skilled in the art. For instance, the examples, embodiments, geometrics, materials, dimensions, ratios, steps, and the like discussed above are illustrative and are not required. Accordingly, the scope of the present invention is understood not to be limited to the details of structure and operation shown and described in the specification and drawings.

The invention is defined by the following claims.

## Claims

1. A surgical instrument (100), comprising:
(a) an ultrasonic blade assembly (2041) including an ultrasonic blade (2042) and at least a portion of a waveguide (2038), wherein the at least a portion of a waveguide (2038) extends along a longitudinal axis, and wherein the ultrasonic blade (2042) distally extends from the at least the portion of the waveguide (2038);
(b) a sheath (2032) positioned over at least a portion of the ultrasonic blade assembly (2041);
(c) a first mating feature (2040) positioned on the ultrasonic blade assembly (2041);
(d) a distal pipe (2034) extending distally from the sheath (2032), wherein the distal pipe (2034) includes:
a second mating feature (2044) configured to be adhered to the first mating feature (2040); and **characterized in that**:
the distal pipe is an overmolded distal pipe, wherein the distal pipe further includes:
a third mating feature (2074) positioned on a proximal portion of the overmolded distal pipe (2034); and further **characterized by** the surgical instrument further including:
(e) a fourth mating feature (2076), wherein the fourth mating feature (2076) is positioned on a distal portion of the sheath (2032), wherein the first, second, third and fourth mating features (2040, 2044, 2074, 2076) are configured to prevent debris from entering an interior of the surgical instrument when the first and second mating features (2040, 2044) are mated together and the third and fourth mating features (2074, 2076) are mated together.

2. The surgical instrument of claim 1, wherein the first mating feature (2040) includes one or more raised portions.

3. The surgical instrument of claim 2, wherein the second mating feature (2044) includes one or more recessed portions configured to mate with the one or more raised portions of the first mating feature (2040).

4. The surgical instrument of any of claims 1 to 3, wherein the third mating feature (2074) includes a tapered region and the fourth mating feature (2076) includes a complimentary tapered region configured to mate with the tapered region of the third mating feature (2074).

5. The surgical instrument of claim 4, wherein one of the third or fourth mating features (2074, 2076) includes a raised portion and the other of the third or fourth mating feature (2074, 2076) includes a recessed portion, wherein the raised portion is configured to mate with the recessed portion.

6. The surgical instrument of any of claims 1 to 5, wherein at least a portion of the overmolded distal pipe (2034) is positioned within an interior diameter of the sheath (2032).

7. The surgical instrument of claim 6, wherein the first mating feature includes a bore (2140) with a progressively decreasing diameter as the bore (2140) approaches the longitudinal axis, and the second mating feature (2144) is defined by the first mating feature (2140).

8. The surgical instrument of any of claims 1 to 7, further including an insulated pin (2060) configured to secure the sheath (2032) to the ultrasonic blade assembly (2041), wherein the insulated pin (2060) is positioned proximally relative to the first mating feature (2040).

9. The surgical instrument of claim 8, further including an overmolded support (2054) configured to support the ultrasonic blade (2042) within the sheath (2032), wherein the overmolded support (2054) is distally positioned relative to the insulated pin (2060) and proximally relative to the first and second mating feature (2040, 2044).

10. A method of manufacturing a surgical instrument according to any preceding claim, the method comprising:
(a) overmolding the distal pipe (2034) directly upon one of the sheath (2032) or the ultrasonic blade assembly (2041).

11. The method of claim 10, further comprising installing a sacrificial seal (2370) over the ultrasonic blade assembly (2041) before overmolding the distal pipe (2034) directly on the ultrasonic blade assembly (2041).

12. The method of claim 11, further comprising removing the sacrificial seal (2370).

13. The method of claim 10, wherein step (a) comprises overmolding the distal pipe (2034) directly upon the sheath (2032), and the method further comprises: (b) mating a distal portion of the sheath (2032) with a proximal portion of the distal pipe (2034) thereby supporting the distal pipe (2034) and sealing an interior of the surgical instrument.

## Patentansprüche

1. Chirurgisches Instrument (100), umfassend:
(a) eine Ultraschallklingenanordnung (2041), einschließlich einer Ultraschallklinge (2042) und mindestens eines Abschnitts eines Wellenleiters (2038), wobei sich der mindestens eine Abschnitt eines Wellenleiters (2038) entlang einer Längsachse erstreckt und wobei sich die Ultraschallklinge (2042) distal von dem mindestens einen Abschnitt des Wellenleiters (2038) erstreckt;
(b) eine Hülle (2032), die über mindestens einem Abschnitt der Ultraschallklingenanordnung (2041) positioniert ist;
(c) ein erstes Eingriffsmerkmal (2040), das an der Ultraschallklingenanordnung (2041) positioniert ist;
(d) ein distales Rohr (2034), das sich distal von der Hülle (2032) erstreckt, wobei das distale Rohr (2034) einschließt:
ein zweites Eingriffsmerkmal (2044), das konfiguriert ist, um an das erste Eingriffsmerkmal (2040) angefügt zu werden; und
**dadurch gekennzeichnet, dass**:
das distale Rohr ein umspritztes distales Rohr ist, wobei das distale Rohr ferner einschließt:
ein drittes Eingriffsmerkmal (2074), das an einem proximalen Abschnitt des umspritzten distalen Rohrs (2034) positioniert ist; und ferner **dadurch gekennzeichnet, dass** das chirurgische Instrument ferner einschließt:
(e) ein viertes Eingriffsmerkmal (2076), wobei das vierte Eingriffsmerkmal (2076) an einem distalen Abschnitt der Hülle (2032) positioniert ist, wobei das erste, zweite, dritte und vierte Eingriffsmerkmal (2040, 2044, 2074, 2076) konfiguriert sind, um zu verhindern, dass Schmutz in ein Inneres des chirurgischen Instruments eindringt, wenn das erste und das zweite Eingriffsmerkmal (2040, 2044) miteinander in Eingriff gebracht sind und das dritte und das vierte Eingriffsmerkmal (2074, 2076) miteinander in Eingriff gebracht sind.

2. Chirurgisches Instrument nach Anspruch 1, wobei das erste Eingriffsmerkmal (2040) einen oder mehrere erhabene Abschnitte einschließt.

3. Chirurgisches Instrument nach Anspruch 2, wobei das zweite Eingriffsmerkmal (2044) einen oder mehrere vertiefte Abschnitte einschließt, die konfiguriert sind, um mit dem einen oder den mehreren erhabenen Abschnitten des ersten Eingriffsmerkmals (2040) in Eingriff gebracht zu werden.

4. Chirurgisches Instrument nach einem der Ansprüche 1 bis 3, wobei das dritte Eingriffsmerkmal (2074) einen konischen Bereich einschließt und das vierte Eingriffsmerkmal (2076) einen komplementären konischen Bereich einschließt, der konfiguriert ist, um mit dem konischen Bereich des dritten Eingriffsmerkmals (2074) in Eingriff gebracht zu werden.

5. Chirurgisches Instrument nach Anspruch 4, wobei eines des dritten oder des vierten Eingriffsmerkmals (2074, 2076) einen erhabenen Abschnitt einschließt und das andere des dritten oder des vierten Eingriffsmerkmals (2074, 2076) einen vertieften Abschnitt einschließt, wobei der erhabene Abschnitt konfiguriert ist, um mit dem vertieften Abschnitt in Eingriff gebracht zu werden.

6. Chirurgisches Instrument nach einem der Ansprüche 1 bis 5, wobei mindestens ein Abschnitt des umspritzten distalen Rohrs (2034) innerhalb eines Innendurchmessers der Hülle (2032) positioniert ist.

7. Chirurgisches Instrument nach Anspruch 6, wobei das erste Eingriffsmerkmal eine Bohrung (2140) mit einem sich bei Annäherung der Bohrung (2140) an die Längsachse progressiv verringernden Durchmesser einschließt und das zweite Eingriffsmerkmal (2144) durch das erste Eingriffsmerkmal (2140) definiert ist.

8. Chirurgisches Instrument nach einem der Ansprüche 1 bis 7, ferner einschließlich eines isolierten Stifts (2060), der konfiguriert ist, um die Hülle (2032) an der Ultraschallklingenanordnung (2041) zu fixieren, wobei der isolierte Stift (2060) proximal relativ zu dem ersten Eingriffsmerkmal (2040) positioniert ist.

9. Chirurgisches Instrument nach Anspruch 8, ferner einschließlich einer umspritzten Stütze (2054), die konfiguriert ist, um die Ultraschallklinge (2042) innerhalb der Hülle (2032) zu stützen, wobei die umspritzte Stütze (2054) distal relativ zu dem isolierten Stift (2060) und proximal relativ zu dem ersten und dem zweiten Eingriffsmerkmal (2040, 2044) positioniert ist.

10. Verfahren zum Herstellen eines chirurgischen Instruments nach einem der vorstehenden Ansprüche, das Verfahren umfassend:
(a) Umspritzen des distalen Rohrs (2034) direkt auf einer der Hülle (2032) oder der Ultraschallklingenanordnung (2041).

11. Verfahren nach Anspruch 10, ferner umfassend ein Installieren einer Opferdichtung (2370) über der Ultraschallklingenanordnung (2041) vor dem Umspritzen des distalen Rohrs (2034) direkt auf der Ultraschallklingenanordnung (2041).

12. Verfahren nach Anspruch 11, ferner umfassend ein Entfernen der Opferdichtung (2370).

13. Verfahren nach Anspruch 10, wobei Schritt (a) das Umspritzen des distalen Rohrs (2034) direkt auf der Hülle (2032) umfasst und das Verfahren ferner umfasst: (b) Ineingriffbringen eines distalen Abschnitts der Hülle (2032) mit einem proximalen Abschnitt des distalen Rohrs (2034), wobei dadurch das distale Rohr (2034) gestützt wird und ein Inneres des chirurgischen Instruments abgedichtet wird.

## Revendications

1. Instrument chirurgical (100), comprenant :
(a) un ensemble lame à ultrasons (2041) comportant une lame à ultrasons (2042) et au moins une partie d'un guide d'ondes (2038), dans lequel l'au moins une partie d'un guide d'ondes (2038) s'étend le long d'un axe longitudinal, et dans lequel la lame à ultrasons (2042) s'étend de manière distale à partir de l'au moins une partie du guide d'ondes (2038) ;
(b) une gaine (2032) positionnée sur au moins une partie de l'ensemble lame à ultrasons (2041) ;
(c) une première caractéristique d'accouplement (2040) positionnée sur l'ensemble lame à ultrasons (2041) ;
(d) un tuyau distal (2034) s'étendant de manière distale à partir de la gaine (2032), dans lequel le tuyau distal (2034) comporte :
une deuxième caractéristique d'accouplement (2044) conçue pour être amenée à adhérer à la première caractéristique d'accouplement (2040) ; et **caractérisé en ce que** :
le tuyau distal est un tuyau distal surmoulé, dans lequel le tuyau distal comporte en outre :
une troisième caractéristique d'accouplement (2074) positionnée sur une partie proximale du tuyau distal surmoulé (2034) ; et **en outre caractérisé en ce que** l'instrument chirurgical comporte en outre :
(e) une quatrième caractéristique d'accouplement (2076), dans lequel la quatrième caractéristique d'accouplement (2076) est positionnée sur une partie distale de la gaine (2032), dans lequel les première, deuxième, troisième et quatrième caractéristiques d'accouplement (2040, 2044, 2074, 2076) sont conçues pour empêcher des débris de pénétrer à l'intérieur de l'instrument chirurgical lorsque les première et deuxième caractéristiques d'accouplement (2040, 2044) sont accouplées ensemble et les troisième et quatrième caractéristiques d'accouplement (2074, 2076) sont accouplées ensemble.

2. Instrument chirurgical selon la revendication 1, dans lequel la première caractéristique d'accouplement (2040) comporte une ou plusieurs parties surélevées.

3. Instrument chirurgical selon la revendication 2, dans lequel la deuxième caractéristique d'accouplement (2044) comporte une ou plusieurs parties en retrait conçues pour s'accoupler avec la ou les parties surélevées de la première caractéristique d'accouplement (2040).

4. Instrument chirurgical selon l'une quelconque des revendications 1 à 3, dans lequel la troisième caractéristique d'accouplement (2074) comporte une région effilée et la quatrième caractéristique d'accouplement (2076) comporte une région effilée complémentaire conçue pour s'accoupler avec la région effilée de la troisième caractéristique d'accouplement (2074).

5. Instrument chirurgical selon la revendication 4, dans lequel l'une des troisième ou quatrième caractéristiques d'accouplement (2074, 2076) comporte une partie surélevée et l'autre de la troisième ou quatrième caractéristique d'accouplement (2074, 2076) comporte une partie en retrait, dans lequel la partie surélevée est conçue pour s'accoupler avec la partie en retrait.

6. Instrument chirurgical selon l'une quelconque des revendications 1 à 5, dans lequel au moins une partie du tuyau distal surmoulé (2034) est positionnée au sein d'un diamètre intérieur de la gaine (2032).

7. Instrument chirurgical selon la revendication 6, dans lequel la première caractéristique d'accouplement comporte un alésage (2140) avec un diamètre progressivement décroissant à mesure que l'alésage (2140) se rapproche de l'axe longitudinal, et la deuxième caractéristique d'accouplement (2144) est définie par la première caractéristique d'accouplement (2140).

8. Instrument chirurgical selon l'une quelconque des revendications 1 à 7, comportant en outre une broche isolée (2060) conçue pour fixer la gaine (2032) à l'ensemble lame à ultrasons (2041), dans lequel la broche isolée (2060) est positionnée de manière proximale par rapport à la première caractéristique d'accouplement (2040).

9. Instrument chirurgical selon la revendication 8, comportant en outre un support surmoulé (2054) conçu pour supporter la lame à ultrasons (2042) à l'intérieur de la gaine (2032), dans lequel le support surmoulé (2054) est positionné de manière distale par rapport à la broche isolée (2060) et de manière proximale par rapport à la première et la deuxième caractéristique d'accouplement (2040, 2044).

10. Procédé de fabrication d'un instrument chirurgical selon l'une quelconque revendication précédente, le procédé comprenant :
(a) le surmoulage du tuyau distal (2034) directement sur l'un de la gaine (2032) ou de l'ensemble lame à ultrasons (2041).

11. Procédé selon la revendication 10, comprenant en outre l'installation d'un joint sacrificiel (2370) sur l'ensemble lame à ultrasons (2041) avant le surmoulage du tuyau distal (2034) directement sur l'ensemble lame à ultrasons (2041).

12. Procédé selon la revendication 11, comprenant en outre le retrait du joint sacrificiel (2370).

13. Procédé selon la revendication 10, dans lequel l'étape (a) comprend le surmoulage du tuyau distal (2034) directement sur la gaine (2032), et le procédé comprend en outre : (b) l'accouplement d'une partie distale de la gaine (2032) avec une partie proximale du tuyau distal (2034), permettant ainsi de supporter le tuyau distal (2034) et de sceller un intérieur de l'instrument chirurgical.
